# EUROPEAN PATENT SPECIFICATION

(11) **EP 3 634 490 B1**
(45) Date of publication and mention of the grant of the patent: **21.02.2024**
(21) Application number: 18728712.3
(22) Date of filing: 23.05.2018
(51) Int. Cl.: A61K 47/32, A61K 47/34, A61K 31/216

(54) **IMPLANTABLE DRUG DELIVERY DEVICE**
IMPLANTIERBARES WIRKSTOFFABGABESYSTEM
DISPOSITIF D'ADMINISTRATION DE MÉDICAMENT À IMPLANTER

(30) Priority: 23.05.2017 GB 201708224
(43) Date of publication of application: 15.04.2020
(73) Proprietor: Catalent UK Supply Chain Limited, Nottingham Business Park Nottingham, Nottinghamshire NG8 6PX (GB)
(72) Inventor: RIGBY-SINGLETON, Shellie, Nottingham NG21 OEN (GB); BARKER, Ian, Nottingham Nottinghamshire NG8 6PX (GB); LUK, Shen, Nottingham Nottinghamshire NG8 6PX (GB); PANKAJ LAD, Rajan, Oxfordshire U.K. OX14 2BZ (GB); KUMAR, Sandeep, Oxfordshire OX12 0FZ (GB)
(74) Representative: Schiweck Weinzierl Koch Patentanwälte Partnerschaft mbB
(86) International application number: PCT/GB2018/051407
(87) International publication number: WO 2018/215772

(56) References cited:
- EP-A1- 0 766 538
- EP-A1- 2 476 409
- US-A1- 2003 224 033

## Description

### Field of the invention

The present invention relates to implantable drug delivery devices and their use as a means of achieving controlled release of one or more active pharmaceutical ingredients.

### Background of the invention

Implantable drug delivery devices are known, and are understood to be devices that are implanted in a bodily orifice (e.g. intravaginal implantation, such as an intravaginal ring), or in bodily tissue (e.g. subcutaneous implantation). Administering a drug by way of an implantable device can offer benefits over oral administration, particularly when looking to achieve more localised drug delivery in order to minimise undesirable systemic effects. Alternative means of achieving more localised drug delivery using non-implantable devices include transdermal patches and topical gels. However, transdermal patches and topical administration can lead to problems such as skin irritation, resulting in discontinuation of treatment (Cohn, Therapeutic Advances in Urology, 2016, Vol. 8(2) 83-90).

Although implantable devices hold the above benefits, controlling the release of the active agent from the implantable device can be problematic, particularly when looking to achieve steady sustained release over an extended period of time. Ideal controlled release is when the initial amount of drug released corresponds to the therapeutic dose, and this dose is then maintained by the delivery system. This release of the drug from the delivery system at a constant rate is referred to as "zero-order release". A particular problem with prior art implantable devices is that a high initial burst of the drug is released. This initially excessive release can cause a number of undesirable drug-related side effects. Following this initial burst release, there is then a rapid drop in release rate, rather than a sustained and steady release.

Existing efforts to minimise the initial burst release include fractionating the drug core of the implantable device into segments. For example, in the prior art are intravaginal rings with a segmented drug core (WO2004096151, and Malcolm et al., J Controlled Release 91 (2003) 465-476). Also in the prior art are intravaginal rings where the drug core is fractionated into pockets (WO2004096151). However, the manufacture of a fractionated core can be challenging, for example due to the complexity of joining the segments together.

US 2003/224033 relates to implantable or insertable medical devices, which comprises: (a) a biocompatible polymer; and (b) at least one therapeutic agent selected from an anti-inflammatory agent, an analgesic agent, an anesthetic agent, and an antispasmodic agent. The medical devices are adapted for implantation or insertion at a site associated with pain or discomfort upon implantation or insertion. In many embodiments, the therapeutic will be selected from at least one of (i) ketorolac and pharmaceutically acceptable salts thereof (e.g., ketorolac tromethamine) and (ii) 4-diethylamino-2-butynylphenylcyclohexyl glycolate and pharmaceutically acceptable salts thereof (e.g., oxybutynin chloride). Also provided are uses for the implantable or insertable medical devices, which uses comprise reducing pain or discomfort accompanying the implantation or insertion of such devices. Further uses may comprise reducing microbial buildup along the device. Methods for manufacturing implantable or insertable medical devices are also provided.

EP 0766538 relates to a non-abusable, non-inflammatory, biocompatible, non-biodegradable, subcutaneous, polymeric implant for the prolonged, controlled release of hydromorphone with near zero-order kinetics. Methods of alleviating cancer pain and treating opioid drug addiction with the implant are also described.

### Summary of the invention

In a first aspect, the presently claimed invention provides an implantable drug delivery device comprising:
an inner core comprising oxybutynin distributed throughout a first water-insoluble polymer, wherein the first water-insoluble polymer is an ethylene vinyl acetate polymer with 10-90 wt% of vinyl acetate, based on the total weight of the ethylene vinyl acetate polymer;
an intermediate coating positioned around the inner core, said intermediate coating comprising an acrylate polymer; and
an outer coating positioned around the intermediate coating, said outer coating comprising a second water-insoluble polymer, wherein the second water-insoluble polymer comprises an ethylene vinyl acetate polymer with 10-90 wt% of vinyl acetate, based on the total weight of the ethylene vinyl acetate polymer;
wherein the acrylate polymer is formed from one or more monomers of formula (I): 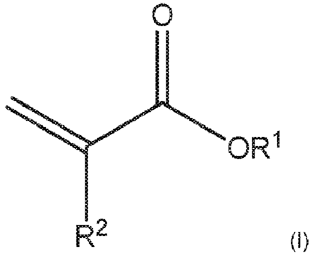
wherein R¹ is selected from H, alkyl, alkenyl, alkynyl, or aryl; and R² is selected from H or alkyl.

In some embodiments, R¹ is selected from alkyl, alkenyl, alkynyl, or aryl, preferably wherein R¹ is alkyl, more preferably C₁-C₄ alkyl.

In some embodiments, the acrylate polymer is a co-polymer, and the one or more monomers comprises a first monomer wherein R¹ is ethyl, and a second monomer wherein R¹ is methyl.

In some embodiments, R² is selected from H or C₁-C₄ alkyl; preferably, R² is selected from H or methyl.

In some embodiments, the acrylate polymer is a co-polymer, and the one or more monomers comprise a first monomer wherein R² is H, and a second monomer wherein R² is alkyl, preferably C₁-C₄ alkyl, more preferably methyl;
Preferably, the acrylate polymer is a co-polymer of ethyl acrylate and methyl methacrylate.

In some embodiments, the thickness of the intermediate coating is at least 10 µm and/or less than 150 µm.

In some embodiments, the thickness of the outer coating is at least 10 µm and/or less than 250 µm.

In some embodiments, the intermediate coating is in direct contact with the inner core, and the outer coating is in direct contact with the intermediate coating.

In some embodiments, the device is an intravaginal ring or a subcutaneous implant.

In some embodiments, the total amount of the at least one pharmaceutical ingredient is at least 2 wt%, preferably at least 5 wt%, and/or is less than 50 wt%, preferably less than 30 wt%, based on the total weight of the inner core.

The presently claimed invention also relates to oxybutynin for use in treating overactive bladder syndrome, wherein the oxybutynin is delivered via the device according to the presently claimed invention.

The presently claimed invention is defined in the appended claims. Preferred embodiments of the presently claimed invention are the subject-matters of the dependent claims and are described throughout the present description and Figures.

Also disclosed herein is an implantable drug delivery device comprising:
an inner core comprising one or more active pharmaceutical ingredients distributed throughout a first water-insoluble polymer;
an intermediate coating positioned around the inner core, said intermediate coating comprising an acrylate polymer; and
an outer coating positioned around the intermediate coating, said outer coating comprising a second water-insoluble polymer;
wherein the acrylate polymer is formed from one or more monomers of formula (I): 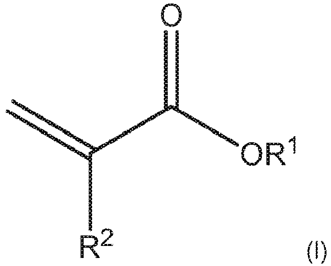
wherein R¹ is selected from H, alkyl, alkenyl, alkynyl, or aryl; and
R² is selected from H or alkyl.

It has been found that an implantable drug delivery device including the above features provides a reduction in the initial drug burst, resulting in improved controlled release. The implantable drug delivery device disclosed herein delivers the active agent continuously, with generally linear release kinetics, in a controlled manner and can be used for treating medical conditions for extended periods of time, such as several days, weeks or months as desired. Without wishing to be bound by theory, it is thought that the water-insoluble polymer of the outer coating (i.e. the second water-insoluble polymer) acts as a protective barrier that, over the duration of use, prevents moisture ingress without impeding permeation of the drug, allowing the acrylate polymer of the intermediate coating to control the rate of release of the drug from the inner core. A further benefit associated with this reduction in initial drug burst is that a lower drug loading is possible, which results in material cost savings and reduced drug wastage.

The acrylate polymer of the intermediate coating provides additional benefits as it adheres well to the inner core, such that it may be easily applied, allowing for straightforward manufacture of the implantable device. This is in contrast to silicone-based polymer coatings, which are cured onto the inner core at a high temperature - this can lead to poor adhesion and/or damage to the materials of the inner core.

The drug delivery device disclosed herein also has suitable mechanical properties e.g. good flexibility, allowing for convenient implantation.

In a second aspect, the presently claimed invention provides oxybutynin for use in treating overactive bladder syndrome, wherein the oxybutynin is delivered via the device according to the presently claimed invention. The implantable drug delivery device is particularly well-adapted for delivering oxybutynin to treat overactive bladder syndrome, owing to the importance of achieving localised and controlled release in this context.

### Description of the preferred embodiments

As used herein, the one or more active pharmaceutical ingredients comprised by the device disclosed herein are understood to include all enantiomers, diastereomers, racemates and mixtures thereof. The drug can be a salt, a solvate, a hydrate, a pro-drug, a co-crystal, a derivative, in free base form, or a mixture thereof.

As used herein, the term "polymer" takes its usual definition the art and so refers to a homopolymer or copolymer formed from the polymerisation of one or more monomers.

As used herein, the term "homopolymer" takes its usual definition in the art, and so refers to a polymer whose polymer chains comprise one type of monomer. As used herein, the term "co-polymer" takes its usual definition in the art, and so refers to a polymer whose polymer chains comprise two or more different types of monomers.

As used herein, the term "monomer" takes its usual definition in the art and so refers to a molecular compound that may chemically bind to another monomer to form a polymer.

As used herein, the term "water-insoluble polymer" refers to polymers wherein less than 10 mg of the polymer dissolves in 100 ml water at 25 °C. This definition applies to both the first water-insoluble polymer (i.e. the water-insoluble polymer of the inner core) and the second water-insoluble polymer (i.e. the water-insoluble polymer of the outer coating) of the implantable drug delivery device.

As used herein, an "implantable drug delivery device" takes its usual definition in the art, and refers to a drug delivery device that is implanted in a bodily orifice or in bodily tissue. Implantable drug delivery devices that are implanted in a bodily orifice include intravaginal implants, rectal implants, and urethral implants. Implantable drug delivery devices that are implanted in bodily tissue include subcutaneous implants, intravascular implants, intraocular implants, intrathecal implants, peritoneal implants, intravesical implants, and intrauterine implants.

Preferably, the implantable drug delivery device is an intravaginal implant or a subcutaneous implant. These implantable drug delivery devices typically remain in-situ for a more prolonged period (e.g. for over 28 days) in comparison to other implantable devices. The drug delivery system disclosed herein is capable of providing controlled release over extended periods of time (e.g. over 28 days). As such the drug delivery system disclosed herein is particularly well-suited for an intravaginal implant or a subcutaneous implant. More preferably, the implantable drug delivery device is an intravaginal ring. Intravaginal rings are adapted for delivery of an active agent to the cervical region. The mechanical properties of the drug delivery system mean it is particularly well-suited to intravaginal rings.

The inner core of the implantable drug delivery device disclosed herein comprises one or more active pharmaceutical ingredients distributed throughout a first water-insoluble polymer. The first water-insoluble polymer, together with the one or more pharmaceutical ingredients, forms the majority of the inner core. The first water-insoluble polymer, together with the one or more pharmaceutical ingredients, can also form the entirety of the inner core.

As used herein, the term "distributed throughout a first water-insoluble polymer" means that the one or more active pharmaceutical ingredient is dissolved or dispersed homogenously throughout the first water-insoluble polymer. This relationship between the one or more active pharmaceutical ingredients and the water-insoluble polymer of the inner core (i.e. the first water-insoluble polymer) may be thought of as a drug-polymer matrix. By encapsulating the one or more active pharmaceutical ingredients within a polymer matrix, the drug may be released as desired from the inner core during normal use, but in the event of the device fracturing, any leakage of the drug(s) from the core is minimised.

The reduced drug burst and improved controlled release is not dependent on fractionating the inner core of the implantable device into segments. However, the device is still compatible with having a segmented inner core. When the inner core is segmented, the inner core comprises multiple segments, each segment of the inner core comprising a water-insoluble polymer, which may be same or different with respect to the water-insoluble polymer of neighbouring segments, and the one or more active pharmaceutical ingredients are distributed throughout the water-insoluble polymer of at least one of the segments. Where there are multiple active pharmaceutical ingredients present in this context, these may be located in the same or in different segments.

Preferably, the inner core is not fractionated into multiple segments, as this further simplifies the manufacturing process of the device. Therefore, the implantable drug delivery device preferably comprises a unitary inner core comprising one or more active pharmaceutical ingredients distributed throughout a first water-insoluble polymer. As used herein, the term "unitary" when used in the context of the inner core refers to the core being of a single segment.

Suitable water-insoluble polymers for the inner core include modified polyolefins (such as ethylene vinyl acetate) polyurethanes, cross-linked silicones, polyesters, polyamides, polyethers, cellulose, and water-insoluble derivatives of cellulose. According to the presently claimed invention, the water-insoluble polymer of the inner core is ethylene vinyl acetate with 10 to 90 wt% of vinyl acetate, based on the total weight of the ethylene vinyl acetate polymer. Using an ethylene vinyl acetate polymer with 10 to 90 wt% of vinyl acetate provides good homogenous distribution of active pharmaceutical ingredient throughout the water-insoluble polymer of the inner core. Preferably, the water-insoluble polymer of the inner core is ethylene vinyl acetate with 15-45 wt% of vinyl acetate, based on the total weight of the ethylene vinyl acetate polymer. In a particularly preferred embodiment, the water-insoluble polymer of the inner core is an ethylene vinyl acetate polymer with 28 wt% vinyl acetate, based on the total weight of the ethylene vinyl acetate polymer. This gives particularly good results, as can be seen from the examples.

The implantable drug delivery device disclosed herein further comprises an intermediate coating positioned around the inner core. The intermediate coating completely coats the inner core irrespective of the shape of the inner core. Said intermediate coating comprises an acrylate polymer formed from one or more monomers of formula (I): 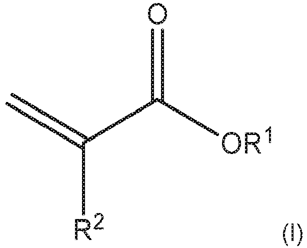
wherein R¹ is selected from H, alkyl, alkenyl, alkynyl, or aryl; and
R² is selected from H or alkyl.

The term "acrylate polymer" when used in its general sense in the art refers to a homopolymer or copolymer formed from one or more monomers that comprise a vinyl group directly bonded to a carboxylic acid/carboxylic ester group. In the context of the present disclosure, the specific acrylate polymers used in the intermediate coating disclosed herein are formed from one or more monomers of formula (I). Preferably, the acrylate polymer of the intermediate coating is a co-polymer.

As used herein, the term "alkyl" refers to C₁-C₁₀ alkyl group, which can be linear or branched. Preferably, alkyl is C₁-C₄ alkyl, such as methyl, ethyl, n-propyl or t-butyl, more preferably methyl or ethyl.

As used herein, the term "alkenyl" refers to C₂-C₁₀ alkenyl group. Preferably, it is a C₂-C₄ alkenyl group. Examples include vinyl, allyl, 1 -propenyl, isopropenyl and 1 -butenyl.

As used herein, the term "alkynyl" refers to a C₂-C₁₀ alkynyl group which can be linear or branched. Preferably, it is a C₂-C₄ alkynyl group.

As used herein, the term "aryl" refers to a monocyclic, bicyclic, or tricyclic aromatic group containing from 1 to 14 carbon atoms in the aromatic system, such as phenyl, biphenyl, naphthyl, anthracenyl, preferably phenyl.

Preferably, R¹ is selected from alkyl, alkenyl, alkynyl, or aryl (where these terms can take any of the above meanings). More preferably R¹ is alkyl, particularly C₁-C₄ alkyl, more preferably methyl or ethyl. In a particularly preferred embodiment, the acrylate polymer is a co-polymer, and the one or more monomers comprise a first monomer wherein R¹ is ethyl, and a second monomer wherein R¹ is methyl.

Preferably, R² is selected from H or C₁-C₄ alkyl, more preferably wherein R² is selected from H or methyl. In a particularly preferred embodiment, the acrylate polymer is a co-polymer, and the one or more monomers comprise a first monomer wherein R² is H, and a second monomer wherein R² is alkyl, preferably C₁-C₄ alkyl, more preferably methyl.

In a particularly preferred embodiment, the acrylate polymer of the intermediate coating is a co-polymer of ethyl acrylate and methyl methacrylate. As the skilled person will appreciate, these monomers have the following structures:

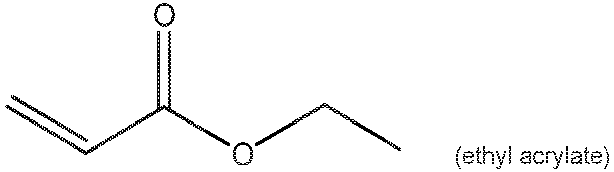

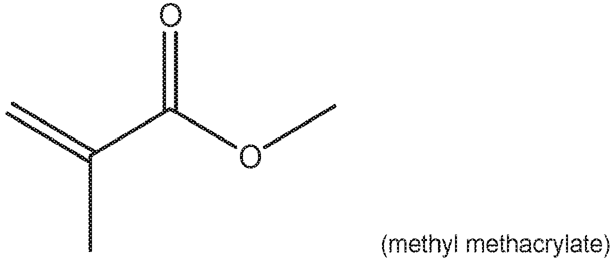

As can be seen from the above structures, in the instance that the acrylate polymer is a co-polymer of ethyl acrylate and methyl methacrylate, the one or more monomers of formula (I) comprise, or consist of, a first monomer wherein R¹ is ethyl and R² is H (ethyl acrylate) and a second monomer wherein R¹ is methyl and R² is methyl (methyl methacrylate).

Most preferably, the acrylate polymer of the intermediate coating is poly(ethyl acrylate, methyl methacrylate), where the ratio of ethyl acrylate : methyl methacrylate is 2 : 1. Such acrylate polymers are available under trade names Eudragit NE 30D or Eudragit NM 30 D. These acrylate polymers provide particularly good reduction in the burst effect and an improved controlled release profile, as can be seen from the examples.

The acrylate polymer forms the majority of the intermediate coating. The acrylate polymer can also form the entirety of the intermediate coating. Preferably, the amount of acrylate polymer in the intermediate coating is at least 80 wt%, more preferably, at least 90 wt%, based on the total weight of the intermediate coating. In addition to the acrylate polymer, the intermediate coating may additionally comprise at least one further component, such as plasticisers, fillers and/or pore forming agents. These additional components are optional, but may impart further benefits. Plasticisers provide flexibility and reduce the possibility and degree of cracking of the acrylate polymer. Plasticisers such as polyethylene glycol, triacetin, diethyl phthalate, dibutyl phthalate, tributyl citrate, triethyl citrate are preferred, as they further improve flexibility without having a detrimental effect on the release profile of the drug. Polyethylene glycol plasticisers are particularly preferred. Fillers, such as talc, provide increased structural rigidity and may be used to enhance the cosmetic appearance of the product.

The implantable drug delivery device disclosed herein further comprises an outer coating positioned around the intermediate coating, said outer coating comprising a second water-insoluble polymer. The outer coating completely coats the intermediate coating irrespective of the shape of the intermediate coating. The second water-insoluble polymer forms the majority of the outer coating. The second water-insoluble polymer can also form the entirety of the outer coating.

Suitable water-insoluble polymers for the outer coating include polyurethanes, ethylene vinyl acetate with 10 to 90 wt% of vinyl acetate based on the total weight of the ethylene vinyl acetate polymer, and cross-linked silicone polymers. According to the presently claimed invention, the water-insoluble polymer of the outer coating comprises or is an ethylene vinyl acetate polymer with 10 to 90 wt% of vinyl acetate, based on the total weight of the ethylene vinyl acetate polymer. Preferably, the water-insoluble polymer of the outer coating is an ethylene vinyl acetate polymer with 20 to 60 wt% of vinyl acetate, based on the total weight of the ethylene vinyl acetate polymer. In a particularly preferred embodiment, the water-insoluble polymer of the outer coating is an ethylene vinyl acetate polymer with 40 wt% of vinyl acetate, based on the total weight of the ethylene vinyl acetate polymer. This gives particularly good results, as can be seen from the examples.

The implantable drug delivery device may further comprise one or more additional layers between the inner core and the intermediate coating, and/or between the intermediate coating and the outer coating. Additional layers may provide certain properties to the device, for example cosmetic enhancement, improved handling of the device, improved mechanical properties and/or improved compression properties. Alternatively, the intermediate coating may be in direct contact with the inner core, and/or the outer coating may be in direct contact with the intermediate coating.

The minimum and maximum thickness of the intermediate coating may be tailored to achieve a particular release rate for the specific drug in question. In terms of the minimum thickness, the thickness of the intermediate coating may be at least 1 µm, at least 10 µm, at least 50 µm, or at least 75 µm. Preferably, the thickness of the intermediate coating is at least 50 µm. In terms of the maximum thickness, the thickness of the intermediate coating may be less than 1000 µm, less than 500 µm, less than 250, or less than 150 µm. Preferably, the thickness of the intermediate coating is less than 100 µm.

The minimum and maximum thickness of the outer coating may be tailored depending on the level of protection that the intermediate coating requires from the in-vivo environment. In terms of the minimum thickness, the thickness of the outer coating may be at least 10 µm, at least 25 µm or at least 50 µm. Preferably, the thickness of the outer coating is at least 10 µm. In terms of the maximum thickness, the thickness of the outer coating is less than 2000 µm, less than 1000 µm, less than 500 µm or less than 250 µm. Preferably, the thickness of the outer coating is less than 50 µm.

The implantable drug delivery device may be used to deliver a variety of one or more active pharmaceutical ingredients. Possible classes of active pharmaceutical ingredients include anticholinergic agents, analgesics, anesthetics, antiarthritics, anti-inflammatory agents, antiasthma drugs, urinary tract disinfectants, anticoagulants, anticonvulsants, antidepressants, antidiabetics, antineoplastics, antipsychotics, antihypertensives, muscle relaxants, antiprotozoals, spermicidals, antibacterials, antihistamines and decongestants, antiparasitic compounds, antiviral compounds, steroidal hormones, opiates, SERMs, SARMs, SPRMs, aromatase inhibitors.

Preferred drug classes include steroidal hormones, opiates, antimuscarinics, SERMs, SARMs, SPRMs and aromatase inhibitors.

Preferred active pharmaceutical ingredients are progesterone, estrogen, estradiol, tamoxifen, raloxifen, lasofoxifene, ospemifen, enobosarm, ulipristal, ulipristal acetate, anastrazole, oxybutynin, toiterodine, trospium, soiifenacin, darifenacin, dicyclomine, propantheline, propiverine, bethanecol, methylbenactyzium and scopolamine, particularly oxybutynin and progesterone.

More preferably, the one or more active pharmaceutical ingredients includes an anticholinergic agent, such as toiterodine, trospium, soiifenacin, darifenacin, dicyclomine, propantheline, propiverine, bethanecol, methylbenactyzium and scopolamine. More preferably, the anticholinergic agent is an antimuscarinic agent, particularly oxybutynin (according to the presently claimed invention).

The implantable drug delivery device is particularly well-suited to delivering one or more active pharmaceutical ingredients with a narrow therapeutic window. As used herein a drug with a "narrow therapeutic window" is a drug where there is a small dosage range between the minimum therapeutic dose and the dose where unwanted side effects are observed. It is particularly important in this context to minimise the initial drug burst and achieve controlled release. As such the active pharmaceutical ingredient can include one or more of the following, which all have a narrow therapeutic window; oxybutynin, isotretinoin, thyroxine, cyclosporine and carbamazepine.

In addition, the active pharmaceutical ingredient preferably includes one or more of the following drugs, which are thought to exhibit suitable diffusion rates from the polymer materials used in the implantable drug delivery device disclosed herein, such that the burst effect is effectively reduced: oxybutynin, fentanyl, buprenorphine, nicotine, diclofenac, ibuprofen, estradiol, progesterone, norgestrel, testosterone, prochlorperazine, rivastigmine, lidocaine, midazolam, and selegiline.

The implantable drug delivery device is particularly well-suited to delivering oxybutynin. Oxybutynin has a narrow therapeutic window and undesirable systemic side effects. Therefore it is particularly important to minimise the initial drug burst and achieve controlled release. The implantable drug delivery device provides especially good results with oxybutynin, as can be seen from the examples. Preferably, the oxybutynin is in free base form.

The implantable drug delivery device may be used to treat a variety of different medical conditions such as overactive bladder syndrome, inflammation, gout, hypercholesterolemia, microbial infection, AIDS, tuberculosis, fungal infection, amoebic infection, parasitic infection, cancer, tumours, organ rejection, diabetes, heart failure, hormonal disorders, arthritis, asthma, pain, congestion, urinary tract infections, vaginal infection, seizure related disorder, depression, psychosis, convulsion, diabetes, blood coagulation and hypertension. The implantable drug delivery device may also be used for birth control i.e. as a contraceptive.

The implantable drug delivery device is well-suited for delivery of a hormone (e.g. as a contraceptive device), as achieving controlled release is particularly desirable in order to minimise any negative systemic effects. The preferable form of the implantable drug delivery device in this context is as a subcutaneous implant.

The implantable drug delivery device is particularly well-suited to treating medical conditions where localised delivery and controlled release are of particular importance. One such condition is overactive bladder syndrome (OAB). More specific OAB conditions include urinary incontinence, urinary urgency, urinary frequency, involuntary bladder contractions and relaxation of the bladder smooth muscle. The preferable form of the implantable drug delivery device in this context is as an intravaginal ring. The skilled person will appreciate that an "intravaginal ring" is a ring shaped device, suitable for placement in the vaginal tract.

Particularly preferred is oxybutynin for use in treating overactive bladder syndrome, wherein the oxybutynin is delivered via the device disclosed herein.

The total amount of the one or more pharmaceutical ingredients present in the inner core may be tailored depending on the therapeutic requirements in question. The total amount of the one or more pharmaceutical ingredients in the inner core is at least 2 wt%, at least 5 wt%, at least 10 wt%, or at least 20 wt%, based on the total weight of the inner core. Preferably, the total amount of the one or more pharmaceutical ingredients in the inner core is at least 5 wt% based on the total weight of the inner core. In terms of the maximum amount, the total amount of the one or more pharmaceutical ingredients in the inner core is less than 50 wt%, less than 40 wt%, or less than 30 wt% based on the total weight of the inner core. Preferably, the total amount of the one or more pharmaceutical ingredients in the inner core is less than 30 wt% based on the total weight of the inner core.

The therapeutically effective dose of the drug that is locally delivered by the device disclosed herein can be tailored depending on the drug in question. Possible dose ranges are from 2 mg/day to 40 mg/day, or from 2 to 10 mg/day. For oxybutynin, the dose range is preferably 2 mg/day to 10 mg/day.

Preferably the device delivers the desired dose of active agent for a period of at least 7 days. More preferably, the device delivers the desired dose of drug for a period of at least 28 days.

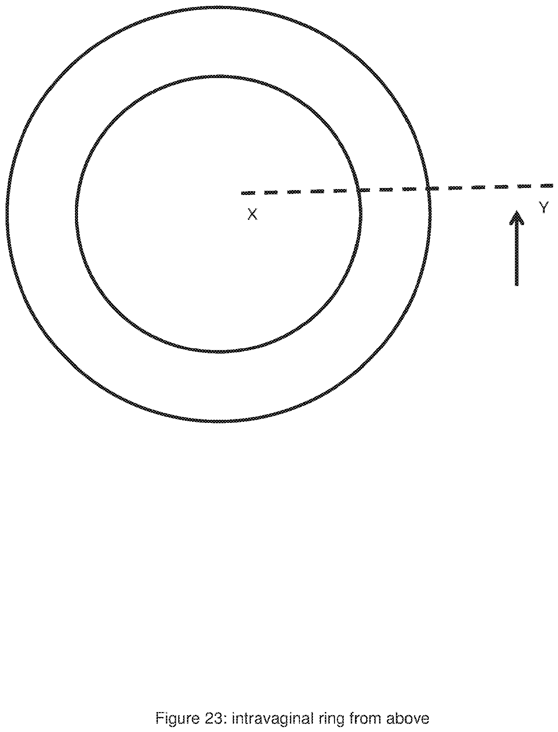

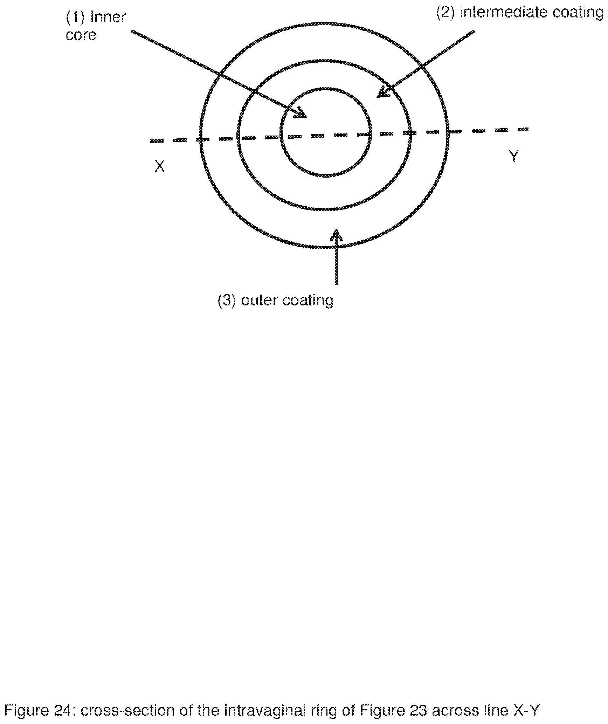

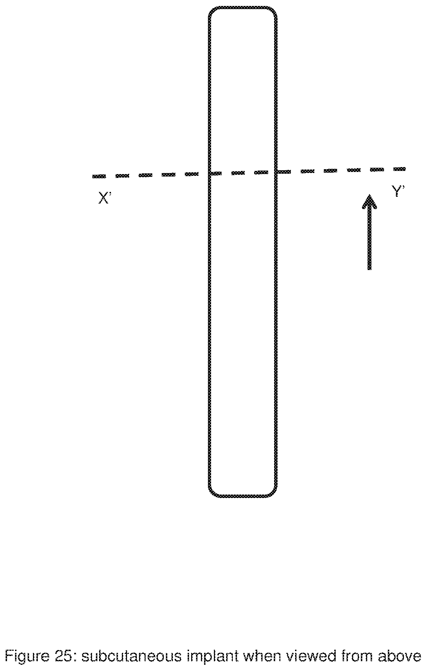

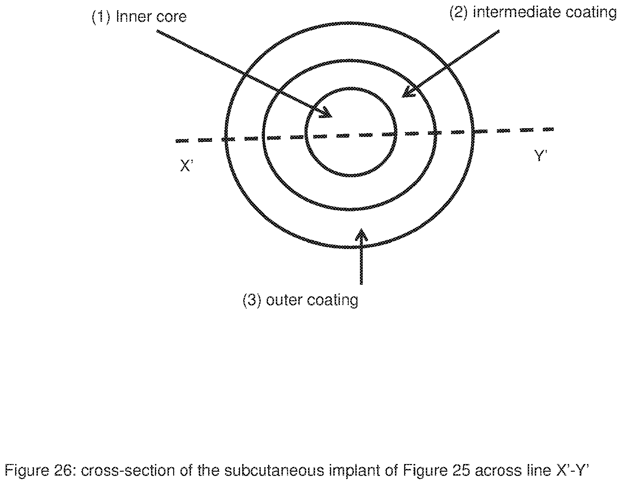

The implantable drug delivery device may take a variety of different shapes. The skilled person will be familiar with suitable shapes that are appropriate for the specific implantable delivery device in question. Possible shapes for an intravaginal ring and a subcutaneous implant are displayed in Figure 23 (intravaginal ring when viewed from above), Figure 24 (cross-section of the intravaginal ring of Figure 23 across line X-Y), Figure 25 (subcutaneous implant when viewed from above) and Figure 26 (cross-section of the subcutaneous implant of Figure 25 across line X'-Y').

The implantable drug delivery device may be manufactured according to a variety of different methods familiar to the skilled person. The inner core may be conveniently manufactured by any conventional moulding technique, for example, by hot melt extrusion of the active agent and core polymer, by injection moulding of the device or by 3-D printing. The intermediate coating may be applied by any coating technique, for example, coextrusion, overmoulding, spray film coating, dip coating and the like. A preferred method involves spray coating of a latex emulsion comprising the intermediate coating onto the inner core or dip coating of the inner core in a dispersion or solution of the intermediate coating. The outer coating may be applied by the same coating methods as those that can be used for applying the intermediate coating, e.g. over-moulding the prefabricated section, or, by way of lamination by hot pressing the device between two layers of the outer coating of the required thickness.

The following examples illustrate the invention.

### Examples

### Comparative Example 1: 10% w/w Oxybutynin-EVA (28% VA content) core.

A 10% w/w blend of Oxybutynin base and EVA (28% VA content) was prepared and hot melt extruded at 110 °C, to produce a solid solution, and pelletised. The pellets were injection moulded using a barrel temperature of 95 °C and a tool temperature of 35 °C to form a ring.

### Comparative Example 2: In-vitro dissolution of Comparative Example 1.

In-vitro dissolution was performed on the intravaginal ring of Comparative Example 1 in 200 mL acetate buffer pH 4.1 at 37 °C, and 100 rpm over a 28 day period using shaking incubator. Dissolution samples were typically taken after 1, 2, 3, 4, 7, 14, 21, 28 days. Dissolution samples were analysed by HPLC.

### Comparative Example 3: 10% w/w Oxybutynin-EVA (28% VA content) core/Eudragit NM 300 coating.

A 10% w/w blend of Oxybutynin base and EVA (28% VA content) was prepared and hot melt extruded at 110 °C, to produce a solid solution, and pelletised. The pellets were injection moulded using a barrel temperature of 95 °C and a tool temperature of 35 °C to form a ring. Eudragit NM 30D solution/dispersion was deposited on the 10% w/w Oxybutynin-EVA core to form a coating with an approximate thickness of 50 - 100 µm.

### Comparative Example 4: In-vitro dissolution of Comparative Example 3.

In-vitro dissolution was performed on the intravaginal ring of Comparative Example 3 in 200 mL acetate buffer pH 4.1 at 37 °C, and 100 rpm over a 28 day period using shaking incubator. Dissolution samples were typically taken after 1, 2, 3, 4, 7, 14, 21, 28 days. Dissolution samples were analysed by HPLC.

### Comparative Example 5: 10% w/wOxybutynin-EVA (28% VA content) core/Eudragit NE 300coating.

A 10% w/w blend of Oxybutynin base and EVA (28% VA content) was prepared and hot melt extruded at 110 °C, to produce a solid solution, and pelletised. The pellets were injection moulded using a barrel temperature of 95 °C and a tool temperature of 35 °C to form a ring. Eudragit NE 30D solution/dispersion was deposited on the 10% w/w Oxybutynin-EVA core to form a coating with an approximate thickness of 50 - 100 µm.

### Comparative Example 6: In-vitro dissolution of Comparative Example 5.

In-vitro dissolution was performed on the intravaginal ring of Comparative Example 5 in 200 mL acetate buffer pH 4.1 at 37 °C, and 100 rpm over a 28 day period using shaking incubator. Dissolution samples were typically taken after 1, 2, 3, 4, 7, 14, 21, 28 days. Dissolution samples were analysed by HPLC.

### Comparative Example 7: 10% w/w Oxybutynin-EVA (28% VA content) core/EVA (40% VA content) coating.

A 10% w/w blend of Oxybutynin base and EVA (28% VA content) was prepared and hot melt extruded at 110 °C, to produce a solid solution, and pelletised. The pellets were injection moulded using a barrel temperature of 95 °C and a tool temperature of 35 °C to form a ring. A solution of EVA (40% VA content) was deposited on the 10% w/w Oxybutynin-EVA core to form a coating with an approximate thickness of 10 - 50 µm.

### Comparative Example 8: In-vitro dissolution of Comparative Example 7.

In-vitro dissolution was performed on the intravaginal ring of Comparative Example 7 in 200 mL acetate buffer pH 4.1 at 37 °C, and 100 rpm over a 28 day period using shaking incubator. Dissolution samples were typically taken after 1, 2, 3, 4, 7, 14, 21, 28 days. Dissolution samples were analysed by HPLC.

### Comparative Example 9: 20%) w/w Oxybutynin-EVA (28% VA content) core

A 20% w/w blend of Oxybutynin base and EVA (28% VA content) was prepared and hot melt extruded at 110 °C, to produce a solid solution, and pelletised. The pellets were injection moulded at using a barrel temperature of 90 °C and a tool temperature of 35 °C to form a ring.

### Comparative Example 10: In-vitro dissolution of Comparative Example 9

In-vitro dissolution was performed on the intravaginal ring of Comparative Example 9 in 200 mL acetate buffer pH 4.1 at 37 °C, and 100 rpm over a 28 day period using shaking incubator. Dissolution samples were typically taken after 1, 2, 3, 4, 7, 14, 21, 28 days. Dissolution samples were analysed by HPLC.

### Example 1: 10% w/w Oxybutynin-EVA (28% VA content) core/Eudragit NM 300 coating/EVA (40% VA content) coating.

A 10% w/w blend of Oxybutynin base and EVA (28% VA content) was prepared and hot melt extruded at 110 °C, to produce a solid solution, and pelletised. The pellets were injection moulded using a barrel temperature of 95 °C and a tool temperature of 35 °C to form a ring. Eudragit NM 30D solution/dispersion was deposited on the 10% w/w Oxybutynin-EVA core to form a coating with an approximate thickness of 50 - 100 µm. A solution of EVA (40% VA content) was deposited on the Eudragit NM 30D coating to form a coating with an approximate thickness of 10 - 50 µm.

### Example 2 In-vitro dissolution of Example 1.

In-vitro dissolution was performed on the intravaginal rings of Example 1 in 200 mL acetate buffer pH 4.1 at 37 °C, and 100 rpm over a 28 day period using shaking incubator. Dissolution samples were typically taken after 1, 2, 3, 4, 7, 14, 21, 28 days. Dissolution samples were analysed by HPLC.

### Example 3: 10% w/w Oxybutynin-EVA (28% VA content) core/Eudragit NE 30D coating/EVA (40% VA content) coating.

A 10% w/w blend of Oxybutynin base and EVA (28% VA content) was prepared and hot melt extruded at 110 °C, to produce a solid solution, and pelletised. The pellets were injection moulded using a barrel temperature of 95 °C and a tool temperature of 35 °C to form a ring. Eudragit NE 30D solution/dispersion was deposited on the 10% w/w Oxybutynin-EVA core to form a coating with an approximate thickness of 50 - 100 µm. A solution of EVA (40%) VA content) was deposited on the Eudragit NE 30D coating to form a coating with an approximate thickness of 10 - 50 µm.

### Example 4 In-vitro dissolution of Example 3.

In-vitro dissolution was performed on the intravaginal ring of Example 3 in 200 mL acetate buffer pH 4.1 at 37 °C, and 100 rpm over a 28 day period using shaking incubator. Dissolution samples were typically taken after 1, 2, 3, 4, 7, 14, 21, 28 days. Dissolution samples were analysed by HPLC.

### Example 5: 20% w/w Oxybutynin-EVA (28% VA content) core/Eudragit NM 300 coating/EVA (40% VA content) coating.

A 20% w/w blend of Oxybutynin base and EVA (28% VA content) was prepared and hot melt extruded at 110 °C, to produce a solid solution, and pelletised. The pellets were injection moulded using a barrel temperature of 90 °C and a tool temperature of 35 °C to form a ring. Eudragit NM 30D solution/dispersion was deposited on the 20% w/w Oxybutynin-EVA core to form a coating with an approximate thickness of 50 - 100 µm. A solution of EVA (40% VA content) was deposited on the Eudragit NM 30D coating to form a coating with an approximate thickness of 10 - 50 µm.

### Example 6 In-vitro dissolution of Example 5.

In-vitro dissolution was performed on the intravaginal ring of Example 5 in 200 mL acetate buffer pH 4.1 at 37 °C, and 100 rpm over a 28 day period using shaking incubator. Dissolution samples were typically taken after 1, 2, 3, 4, 7, 14, 21, 28 days. Dissolution samples were analysed by HPLC.

### Example 7: 20% w/w Oxybutynin-EVA (28% VA content) core/Eudragit NE 30D coating/EVA (40% VA content) coating.

A 20% w/w blend of Oxybutynin base and EVA (28% VA content) was prepared and hot melt extruded at 110 °C, to produce a solid solution, and pelletised. The pellets were injection moulded using a barrel temperature of 90 °C and a tool temperature of 35 °C to form a ring. Eudragit NE 30D solution/dispersion was deposited on the 20% w/w Oxybutynin-EVA core to form a coating with an approximate thickness of 50 - 100 µm. A solution of EVA (40%) VA content) was deposited on the Eudragit NE 30D coating to form a protective coating with an approximate thickness of 10 - 50 µm.

### Example 8 In-vitro dissolution of Example 7.

In-vitro dissolution was performed on the intravaginal ring of Example 7 in 200 mL acetate buffer pH 4.1 at 37 °C, and 100 rpm over a 28 day period using shaking incubator. Dissolution samples were typically taken after 1, 2, 3, 4, 7, 14, 21, 28 days. Dissolution samples were analysed by HPLC.

### Results

### Comparative Example 1

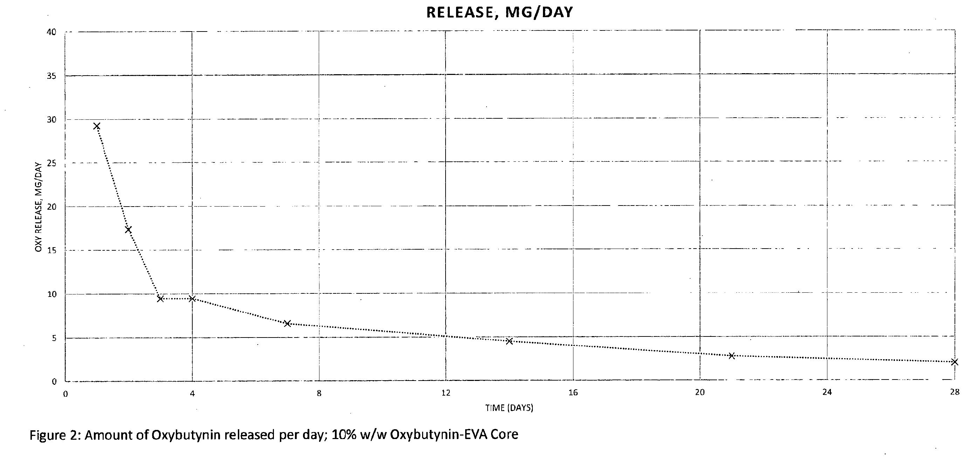

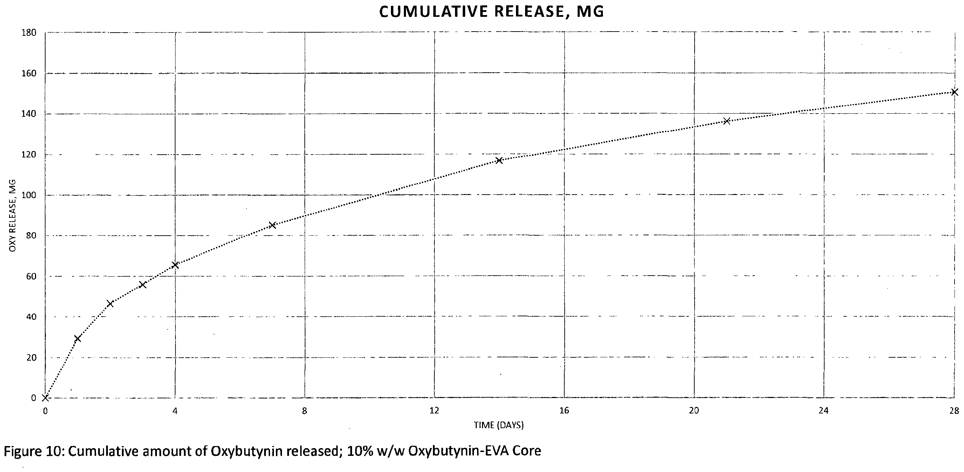

10% w/w Oxybutynin base is molecularly dispersed in ethyl vinyl acetate (28% vinyl acetate content). The in vitro dissolution profile is presented in Figure 2 showing the amount of oxybutynin released per day over a 28 day period, and Figure 10 shows the cumulative amount of oxybutynin released over a 28 day period. A "burst" occurs on day 1 rapidly decreasing thereafter up to day 3 or 4, after which a gradual decrease in the amount of oxybutynin release is released over the 28 day period (Figure 2). The release rate is typical of that of a Higuchi release profile (see Figure 10). This shows that a simple drug in polymer matrix does not achieve the desired zero order release profile.

### Comparative Example 3

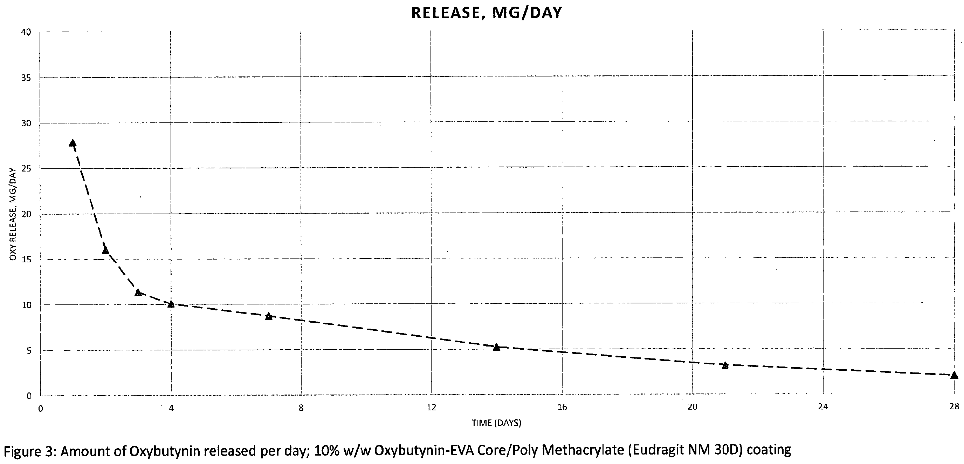

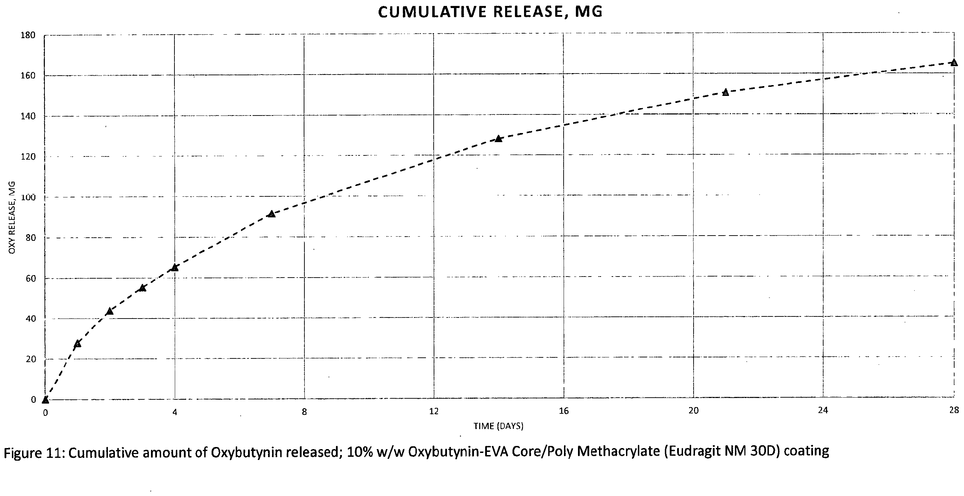

10% w/w Oxybutynin base is molecularly dispersed in ethyl vinyl acetate (28% vinyl acetate content) to produce a core in which Eudragit NM 30D was deposited onto the surface of the core to produce a coating. The in vitro dissolution profile is presented in Figure 3 showing amount of oxybutynin released per day over a 28 day period and Figure 11 showing the cumulative amount of oxybutynin released over a 28 day period. Despite the addition of Eudragit, a "burst" still occurs on day 1 rapidly decreasing thereafter up to day 3 or 4, after which a slower rate of release is observed over the 28 day period (Figure 3). The release rate is typical of that of a Higuchi release profile (Figure 11). This shows that a simple drug in polymer matrix, with the addition of a polymethacrylate polymer coating does not achieve the desired zero order release profile.

### Comparative Example 5

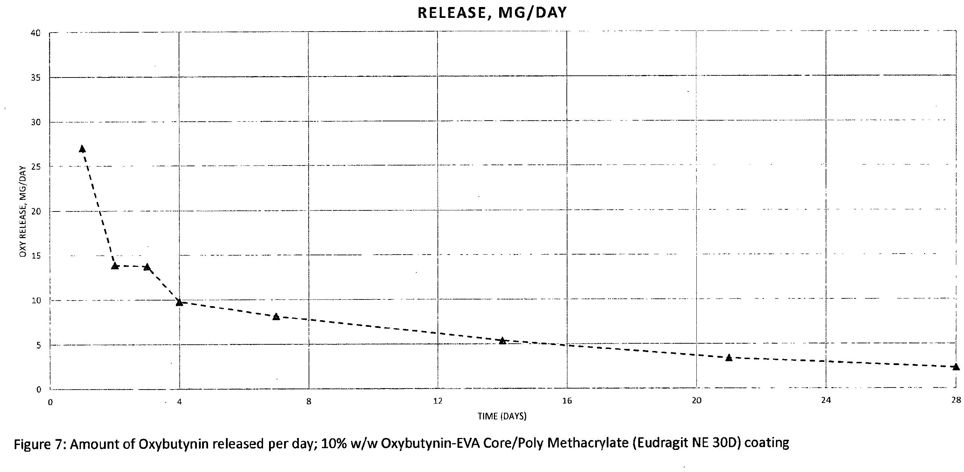

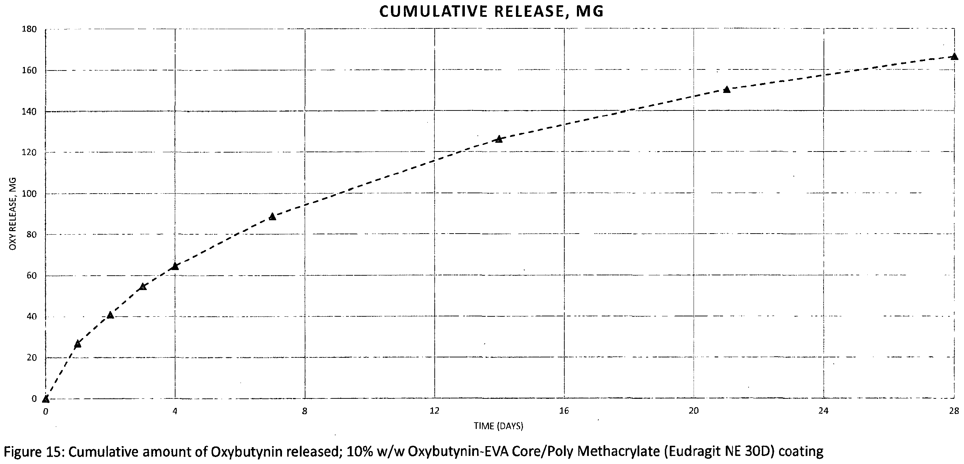

10% w/w Oxybutynin base is molecularly dispersed in ethyl vinyl acetate (28% vinyl acetate content) to produce a core in which Eudragit NE 30D was deposited onto the surface of the core to produce a coating. The in vitro dissolution profile is presented in Figure 7 showing amount of oxybutynin released per day over a 28 day period and Figure 15 showing the cumulative amount of oxybutynin released over a 28 day period. Despite the addition of Eudragit, a "burst" still occurs on day 1 rapidly decreasing thereafter up to day 3 or 4, after which a slower rate of release is observed over the 28 day period (Figure 7). The release rate is typical of that of a Higuchi release profile (Figure 15). This shows that a simple drug in polymer matrix, with the addition of a polymethacrylate polymer coating does not achieve the desired zero order release profile.

### Comparative Example 7

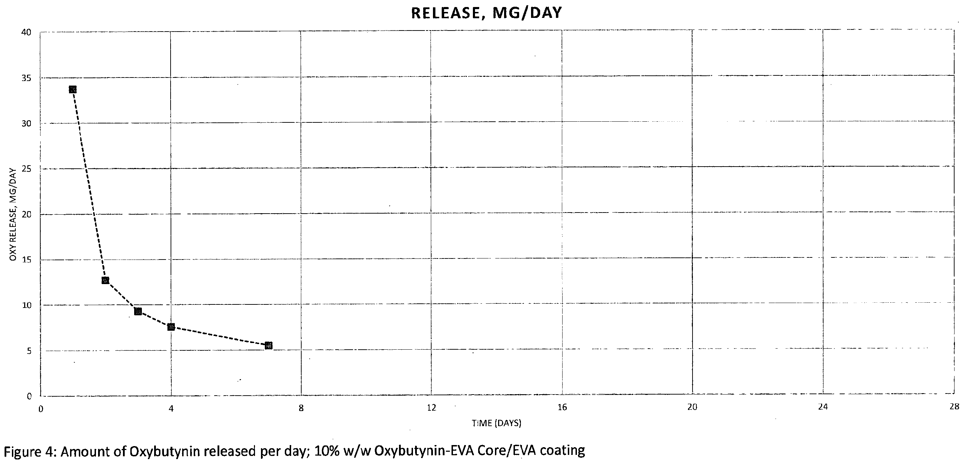

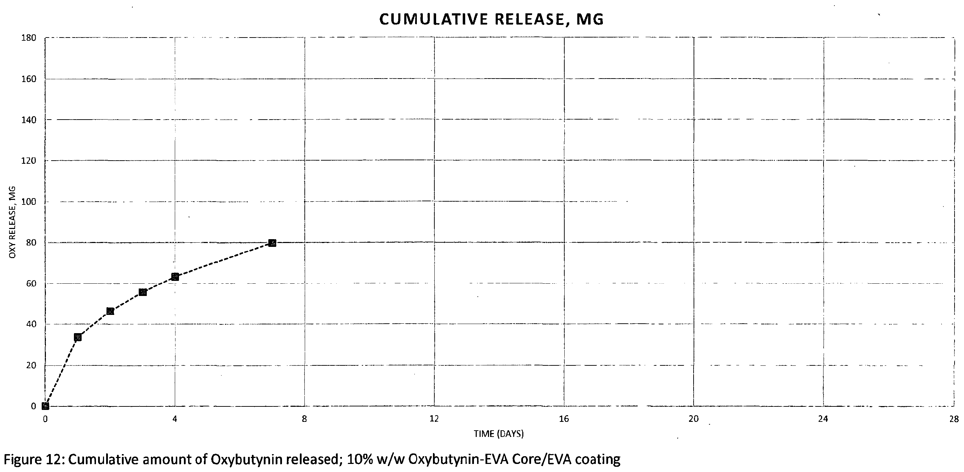

10% w/w Oxybutynin base is molecularly dispersed in ethyl vinyl acetate (28% vinyl acetate content) to produce a core in which ethyl vinyl acetate (40% vinyl acetate content) was deposited onto the surface of the core to produce a coating. The in vitro dissolution profiles is presented in Figure 4 showing amount of oxybutynin released per day over a 28 day period and Figure 12 showing the cumulative amount of oxybutynin released over a 28 day period. A "burst" occurs on day 1 rapidly decreasing thereafter up to day 3 -4, after which a gradual decrease in the amount of oxybutynin release is observed (Figure 4). The release rate is typical of that of a Higuchi release profile (Figure 12). This shows that a simple drug in polymer matrix with the addition of an EVA (40% vinyl acetate content) coating does not achieve the desired zero order release profile.

### Comparative Example 9

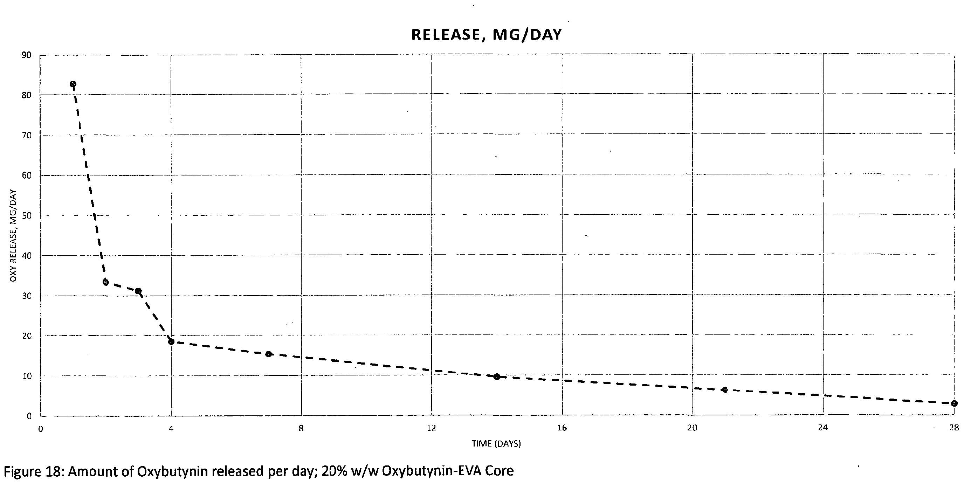

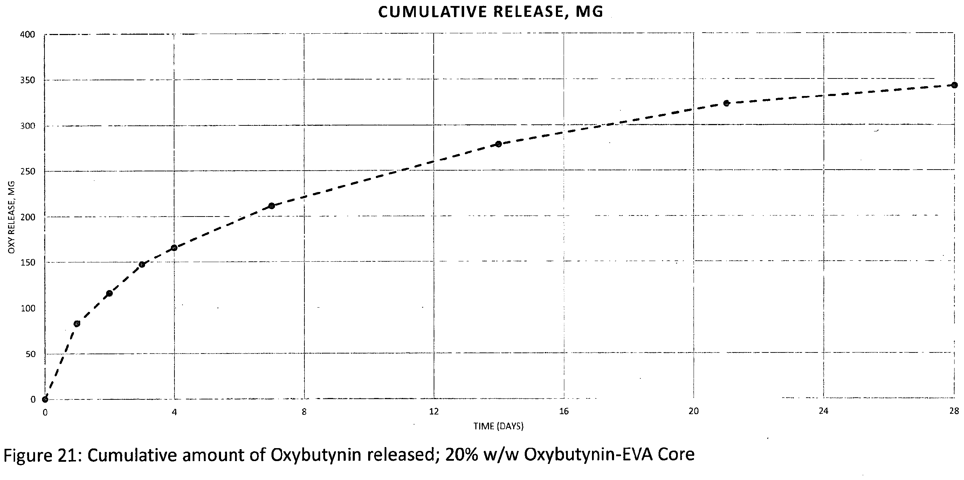

20% w/w Oxybutynin base is molecularly dispersed in ethyl vinyl acetate (28% vinyl acetate content). The in vitro dissolution profile is presented in Figure 18 showing the amount of oxybutynin released per day over a 28 day period and Figure 21 showing the cumulative amount of oxybutynin released over a 28 day period. A "burst" occurs on day 1 rapidly decreasing thereafter followed by a gradual decrease in the amount of oxybutynin released over the 28 day period (Figure 18). The release rate is typical of that of a Higuchi release profile (see Figure 21). This shows that a simple drug in polymer matrix does not achieve the desired zero order release profile.

### Example 1

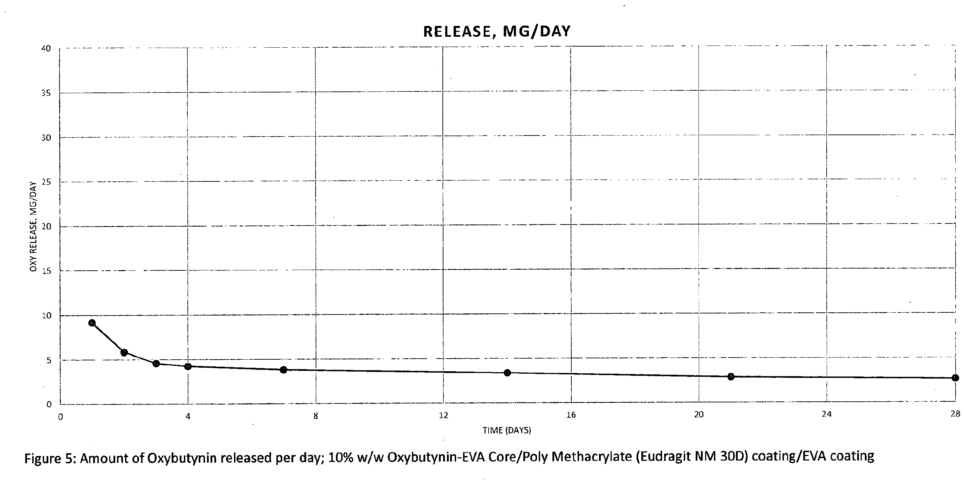

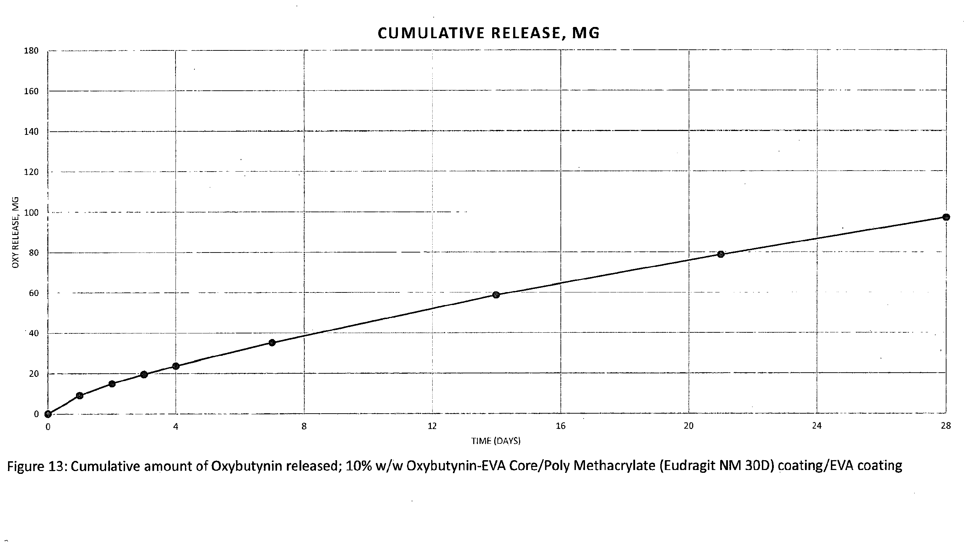

Combines the 10% w/w Oxybutynin base molecularly dispersed in ethyl vinyl acetate (28% vinyl acetate content) core, the Eudragit NM 30D coating and the ethyl vinyl acetate (40% vinyl acetate content) coating, where the 10% w/w oxybutynin in EVA (28% VA content) forms the inner core to which the Eudragit NM 30D coating is deposited, and to the Eudragit NM 30D is deposited EVA (40% VA content) coating. The in vitro dissolution profile is presented in Figure 5 showing amount of oxybutynin released per day over a 28 day period and Figure 13 showing the cumulative amount of oxybutynin released over a 28 day period. These figures show a significant reduction in the "burst" effect with a near constant amount of Oxybutynin release over the 28 day period (Figure 5). The release rate is typical of that of a zero order release profile (Figure 13).

The comparison of these results with Figures 2, 3, and 4 shows that in the absence of either the acrylate polymer coating or the water-insoluble coating this reduction in the burst effect is not achieved.

### Example 3

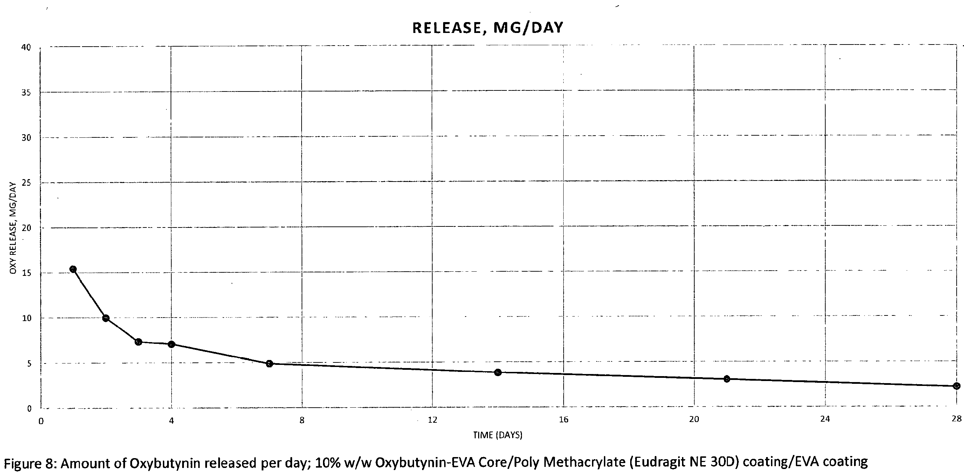

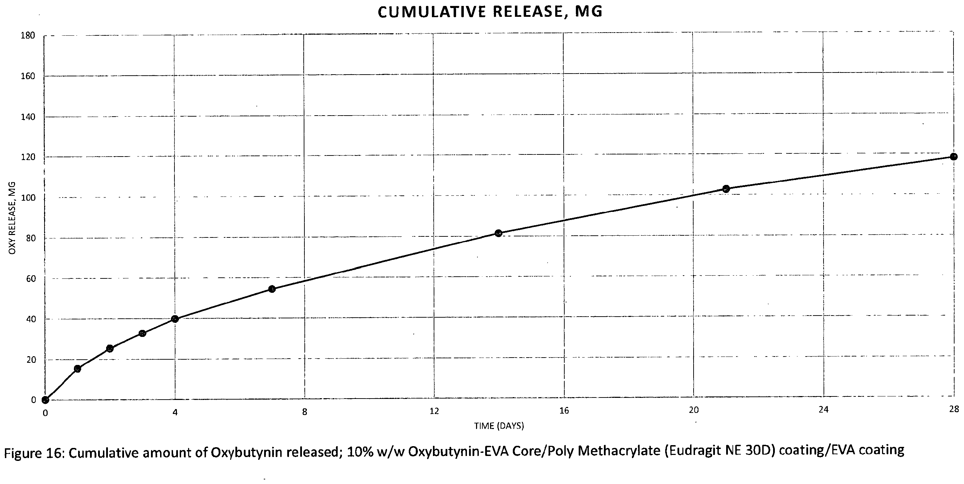

Combines the 10% w/w Oxybutynin base molecularly dispersed in ethyl vinyl acetate (28% vinyl acetate content) core, the Eudragit NE 30D polymer coating and the ethyl vinyl acetate (40% vinyl acetate content) coating, where the 10% w/w oxybutynin in EVA (28% VA content) forms the inner core to which the Eudragit NE 30D coating is deposited, and to the Eudragit NE 30D coating is deposited the ethyl vinyl acetate (40% vinyl acetate content) coating. The in vitro dissolution profile is presented in Figure 8 showing the amount of oxybutynin released per day over a 28 day period and Figure 16 showing the cumulative amount of oxybutynin released over a 28 day period. These figures show a significant reduction in the "burst" effect with a near constant amount of Oxybutynin release over the 28 day period (Figure 8). The release rate is typical of that of a zero order release profile (Figure 16).

The comparison of these results with Figures 2, 3, and 4 shows that in the absence of either the acrylate polymer coating or the water-insoluble coating this reduction in the burst effect is not achieved.

### Example 5

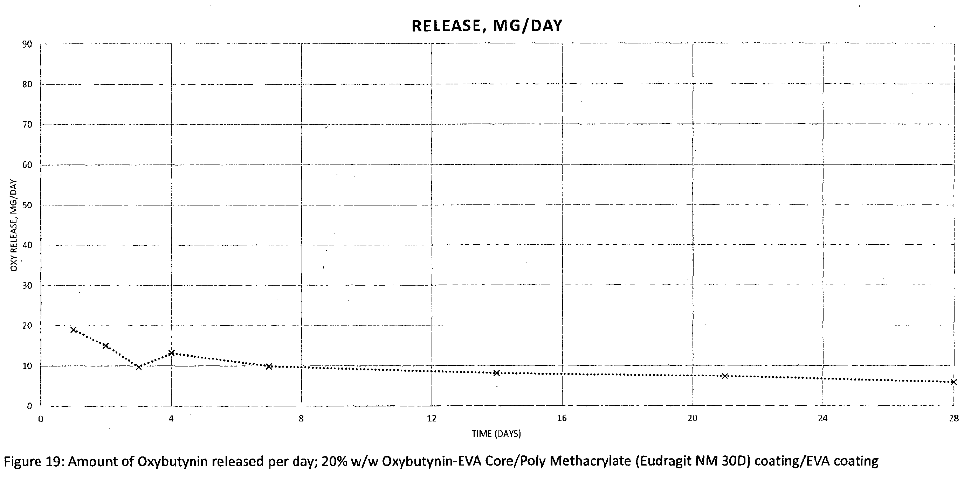

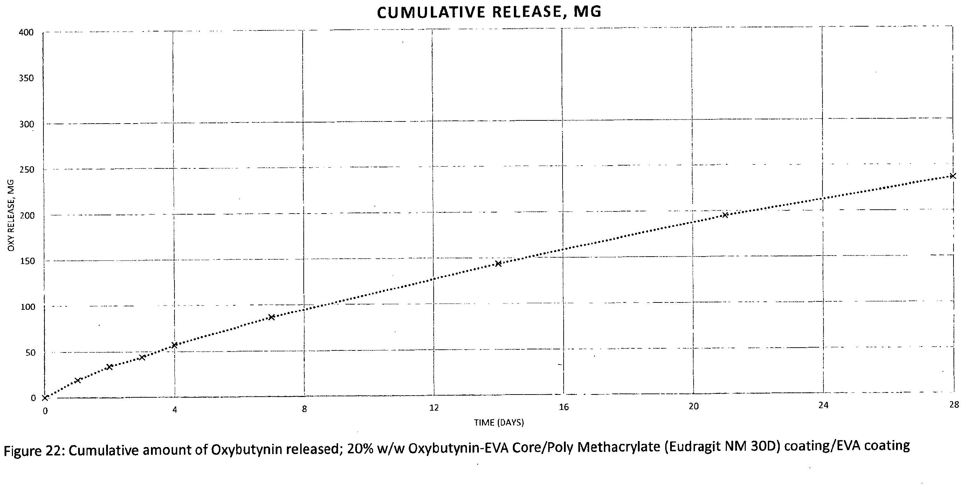

Combines a 20% w/w Oxybutynin base molecularly dispersed in ethyl vinyl acetate (28% vinyl acetate content) core, the Eudragit NM 30D polymer coating, and the ethyl vinyl acetate (40% vinyl acetate content) coating where the 20% w/w oxybutynin in EVA (28% VA content) forms the inner core to which the Eudragit NM 30D coating is deposited, and to the Eudragit NM 30D coating is deposited the EVA (40% VA content) coating. The in vitro dissolution profile is presented in Figure 19 showing the amount of oxybutynin released per day over a 28 day period and Figure 22 showing the cumulative amount of oxybutynin released over a 28 day period. These figures show a significant reduction in the "burst" effect with a near constant amount of Oxybutynin release over the 28 day period (Figure 19). The release rate is typical of that of a zero order release profile (Figure 22).

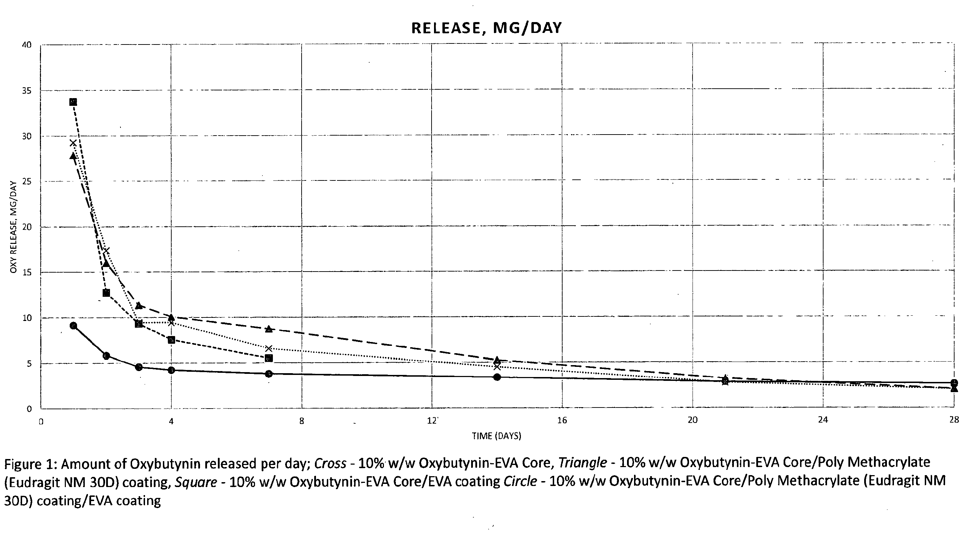

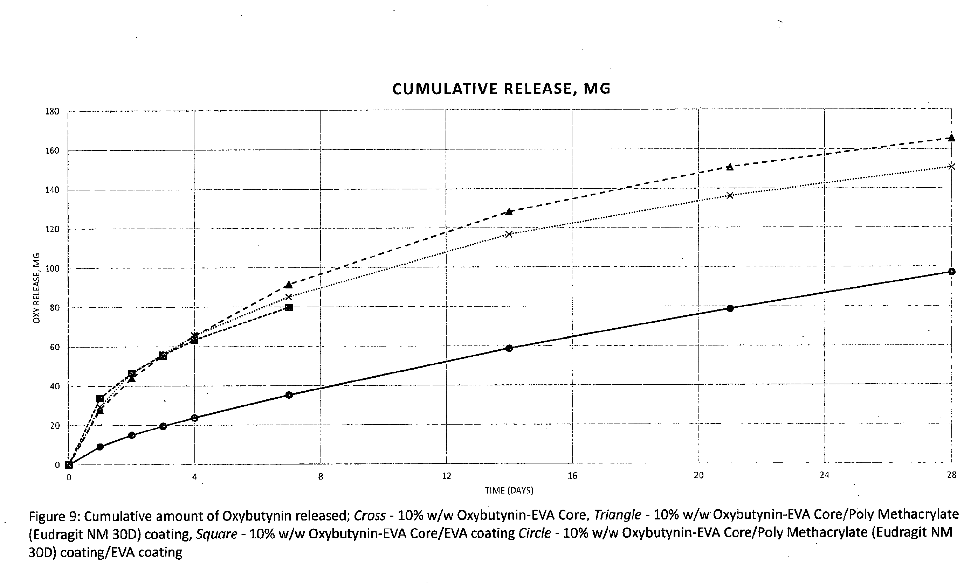

For ease of reference, the in vitro dissolution profiles of comparative example 1, comparative example 3, comparative example 7, and example 1 are displayed on Figure 1 showing the amount of oxybutynin released per day over a 28 day period and on Figure 9 showing the cumulative amount of oxybutynin released over a 28 day period.

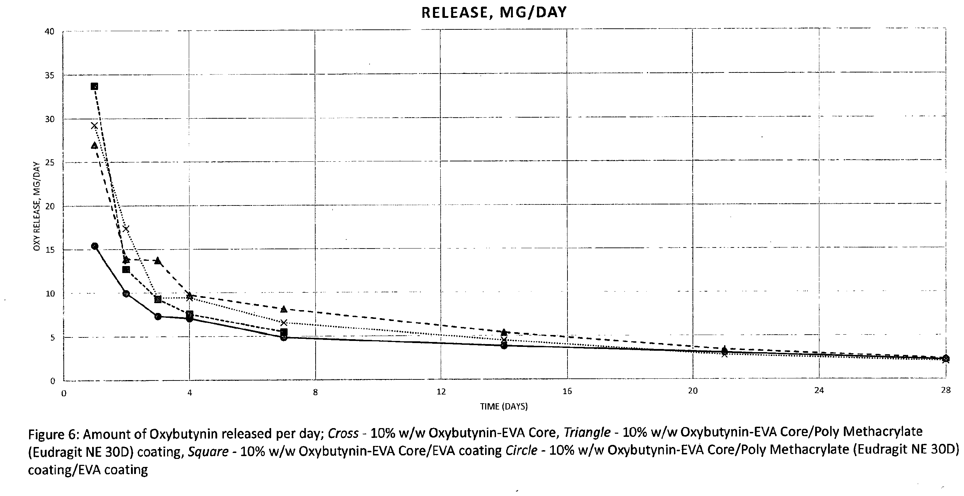

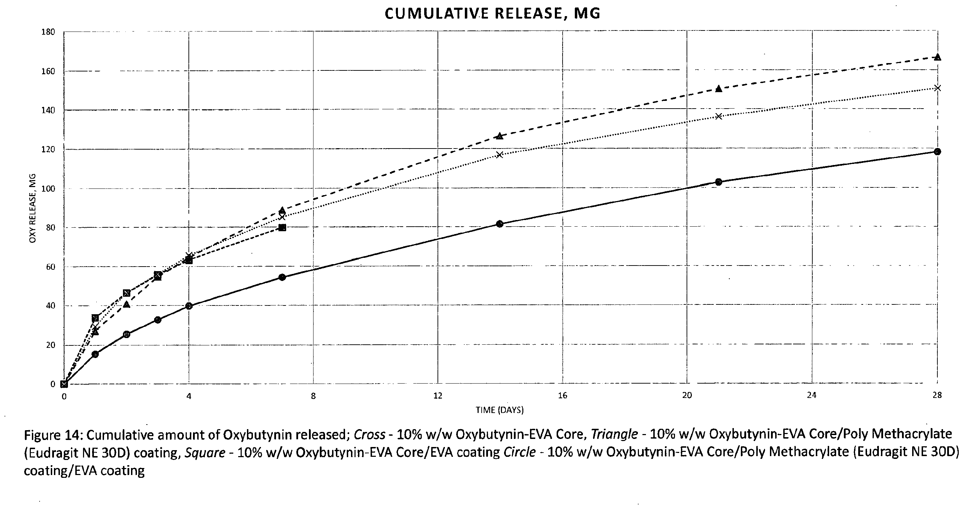

For ease of reference, the in vitro dissolution profiles of comparative example 1, comparative example 5, comparative example 7, and example 3 are displayed on Figure 6 showing the amount of oxybutynin released per day over a 28 day period and on Figure 14 showing the cumulative amount of oxybutynin released over a 28 day period.

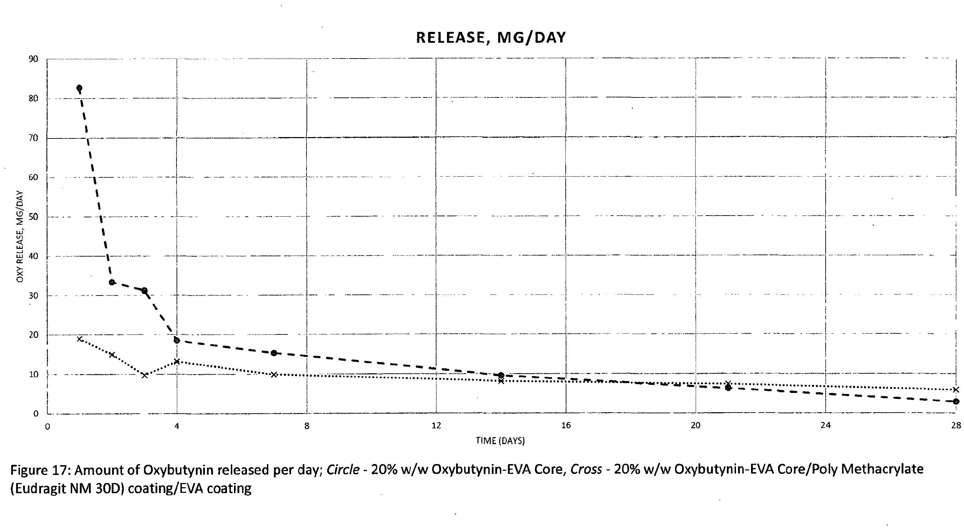

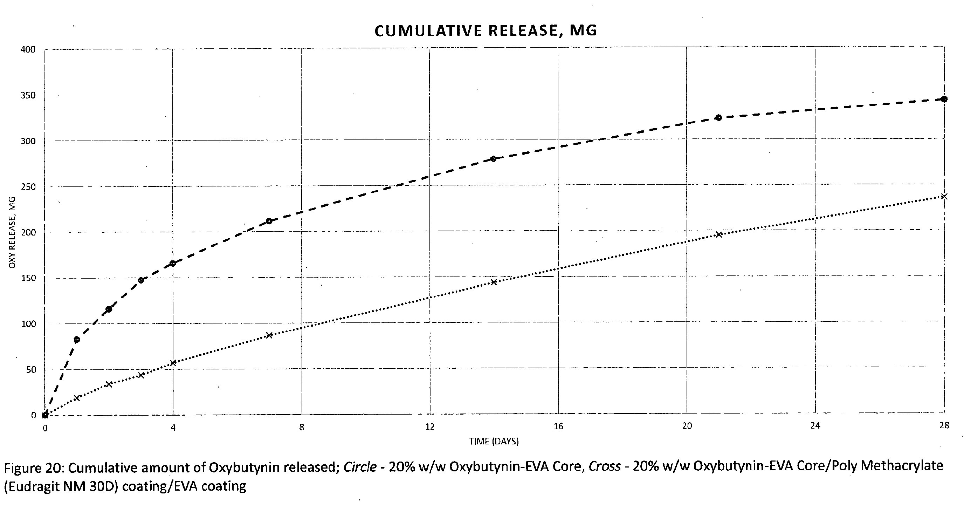

For ease of reference, the in vitro dissolution profiles of comparative example 9 and example 5 are displayed on Figure 17 showing the amount of oxybutynin released per day over a 28 day period and on Figure 20 showing the cumulative amount of oxybutynin released over a 28 day period.

## Claims

1. An implantable drug delivery device comprising:
an inner core comprising oxybutynin distributed throughout a first water-insoluble polymer, wherein the first water-insoluble polymer is an ethylene vinyl acetate polymer with 10-90 wt% of vinyl acetate, based on the total weight of the ethylene vinyl acetate polymer;
an intermediate coating positioned around the inner core, said intermediate coating comprising an acrylate polymer; and
an outer coating positioned around the intermediate coating, said outer coating comprising a second water-insoluble polymer, wherein the second water-insoluble polymer comprises an ethylene vinyl acetate polymer with 10-90 wt% of vinyl acetate, based on the total weight of the ethylene vinyl acetate polymer;
wherein the acrylate polymer is formed from one or more monomers of formula (I): 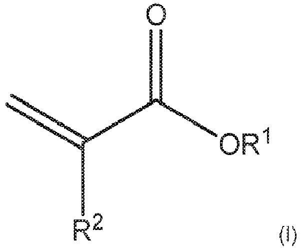
wherein R¹ is selected from H, alkyl, alkenyl, alkynyl, or aryl; and R² is selected from H or alkyl.

2. The device according to any preceding claim, wherein R¹ is selected from alkyl, alkenyl, alkynyl, or aryl, preferably wherein R¹ is alkyl, more preferably C₁-C₄ alkyl.

3. The device according to any preceding claim, wherein the acrylate polymer is a co-polymer, and the one or more monomers comprises a first monomer wherein R¹ is ethyl, and a second monomer wherein R¹ is methyl.

4. The device according to any preceding claim, wherein R² is selected from H or C₁-C₄ alkyl, preferably wherein R² is selected from H or methyl.

5. The device according to any preceding claim, wherein the acrylate polymer is a co-polymer, and the one or more monomers comprise a first monomer wherein R² is H, and a second monomer wherein R² is alkyl, preferably C₁-C₄ alkyl, more preferably methyl; preferably wherein the acrylate polymer is a co-polymer of ethyl acrylate and methyl methacrylate.

6. The device according to any preceding claim, wherein the thickness of the intermediate coating is at least 10 µm and/or less than 150 µm.

7. The device according to any preceding claim, wherein the thickness of the outer coating is at least 10 µm and/or less than 250 µm.

8. The device according to any preceding claim, wherein the intermediate coating is in direct contact with the inner core, and the outer coating is in direct contact with the intermediate coating.

9. The device according to any preceding claim, wherein the device is an intravaginal ring or a subcutaneous implant.

10. The device according to any preceding claim, wherein the total amount of the at least one pharmaceutical ingredient is at least 2 wt%, preferably at least 5 wt%, and/or is less than 50 wt%, preferably less than 30 wt%, based on the total weight of the inner core.

11. Oxybutynin for use in treating overactive bladder syndrome, wherein the oxybutynin is delivered via the device according to any preceding claim.

## Patentansprüche

1. Implantierbare Arzneimittelabgabevorrichtung, umfassend:
einen inneren Kern, umfassend Oxybutynin, das in einem ersten wasserunlöslichen Polymer verteilt ist, wobei das erste wasserunlösliche Polymer ein Ethylen-Vinylacetat-Polymer mit 10 bis 90 Gew.% Vinylacetat ist, basierend auf dem Gesamtgewicht des Ethylen-Vinylacetat-Polymers;
eine Zwischenbeschichtung, die um den inneren Kern herum angeordnet ist, wobei die Zwischenbeschichtung ein Acrylatpolymer umfasst; und
eine äußere Beschichtung, die um die Zwischenbeschichtung herum angeordnet ist, wobei die äußere Beschichtung ein zweites wasserunlösliches Polymer umfasst, wobei das zweite wasserunlösliche Polymer ein Ethylen-Vinylacetat-Polymer mit 10 bis 90 Gew.% Vinylacetat ist, basierend auf dem Gesamtgewicht des Ethylen-Vinylacetat-Polymers;
wobei das Acrylatpolymer aus einem oder mehreren Monomeren der Formel (I): 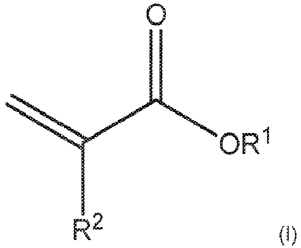
gebildet ist, wobei R¹ aus H, Alkyl, Alkenyl, Alkinyl oder Aryl ausgewählt ist; und R² aus H oder Alkyl ausgewählt ist.

2. Vorrichtung nach einem vorstehenden Anspruch, wobei R¹ aus Alkyl, Alkenyl, Alkinyl oder Aryl ausgewählt ist, vorzugsweise wobei R¹ Alkyl, mehr bevorzugt C₁-C₄-Alkyl ist.

3. Vorrichtung nach einem vorstehenden Anspruch, wobei das Acrylatpolymer ein Copolymer ist und das eine oder die mehreren Monomere ein erstes Monomer, wobei R¹ Ethyl ist, und ein zweites Monomer, wobei R¹ Methyl ist, umfassen.

4. Vorrichtung nach einem vorstehenden Anspruch, wobei R² aus H oder C₁-C₄-Alkyl ausgewählt ist, vorzugsweise wobei R² aus H oder Methyl ausgewählt ist.

5. Vorrichtung nach einem vorstehenden Anspruch, wobei das Acrylatpolymer ein Copolymer ist und das eine oder die mehreren Monomere ein erstes Monomer, wobei R² H ist, und ein zweites Monomer umfassen, wobei R² Alkyl, vorzugsweise C₁-C₄-Alkyl, mehr bevorzugt Methyl ist;
vorzugsweise wobei das Acrylatpolymer ein Copolymer aus Ethylacrylat und Methylmethacrylat ist.

6. Vorrichtung nach einem vorstehenden Anspruch, wobei die Dicke der Zwischenbeschichtung mindestens 10 µm und/oder weniger als 150 µm beträgt.

7. Vorrichtung nach einem vorstehenden Anspruch, wobei die Dicke der äußeren Beschichtung mindestens 10 µm und/oder weniger als 250 µm beträgt.

8. Vorrichtung nach einem vorstehenden Anspruch, wobei die Zwischenbeschichtung in direktem Kontakt mit dem inneren Kern steht und die äußere Beschichtung in direktem Kontakt mit der Zwischenbeschichtung steht.

9. Vorrichtung nach einem vorstehenden Anspruch, wobei die Vorrichtung ein intravaginaler Ring oder ein subkutanes Implantat ist.

10. Vorrichtung nach einem vorstehenden Anspruch, wobei die Gesamtmenge des mindestens einen pharmazeutischen Bestandteils mindestens 2 Gew.%, vorzugsweise mindestens 5 Gew.% und/oder weniger als 50 Gew.%, vorzugsweise weniger als 30 Gew.% beträgt, basierend auf dem Gesamtgewicht des inneren Kerns.

11. Oxybutynin zur Verwendung bei der Behandlung eines Syndroms einer überaktiven Blase, wobei das Oxybutynin über die Vorrichtung nach einem vorstehenden Anspruch abgegeben wird.

## Revendications

1. Dispositif implantable d'administration de médicament comprenant :
un noyau intérieur comprenant de l'oxybutynine distribuée dans un premier polymère insoluble dans l'eau, le premier polymère insoluble dans l'eau étant un polymère d'éthylène-acétate de vinyle avec 10 à 90 % en poids d'acétate de vinyle, par rapport au poids total du polymère d'éthylène-acétate de vinyle ;
un revêtement intermédiaire positionné autour du noyau intérieur, ledit revêtement intermédiaire comprenant un polymère d'acrylate ; et
un revêtement extérieur positionné autour du revêtement intermédiaire, ledit revêtement extérieur comprenant un second polymère insoluble dans l'eau, le second polymère insoluble dans l'eau comprenant un polymère d'éthylène-acétate de vinyle avec 10 à 90 % en poids d'acétate de vinyle par rapport au poids total du polymère d'éthylène-acétate de vinyle ;
dans lequel le polymère d'acrylate est formé à partir d'un ou plusieurs monomères de formule (I) : 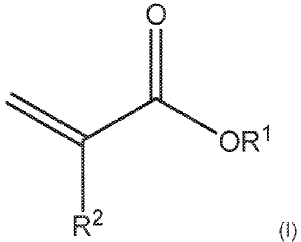
dans laquelle R¹ est choisi parmi H, alkyle, alcényle, alcynyle ou aryle ; et R² est choisi parmi H ou alkyle.

2. Dispositif selon l'une quelconque des revendications précédentes, dans lequel R¹ est choisi parmi alkyle, alcényle, alcynyle ou aryle, de préférence dans lequel R¹ est alkyle, plus préférentiellement alkyle en C₁-C₄.

3. Dispositif selon l'une quelconque des revendications précédentes, dans lequel le polymère d'acrylate est un copolymère, et les un ou plusieurs monomères comprennent un premier monomère dans lequel R¹ est éthyle, et un second monomère dans lequel R¹ est méthyle.

4. Dispositif selon l'une quelconque des revendications précédentes, dans lequel R² est choisi parmi H ou alkyle en C₁-C₄, de préférence dans lequel R² est choisi parmi H ou méthyle.

5. Dispositif selon l'une quelconque des revendications précédentes, dans lequel le polymère d'acrylate est un copolymère, et les un ou plusieurs monomères comprennent un premier monomère dans lequel R² est H, et un second monomère dans lequel R² est alkyle, de préférence alkyle en C₁-C₄, plus préférentiellement méthyle ;
de préférence dans lequel le polymère d'acrylate est un copolymère d'acrylate d'éthyle et de méthacrylate de méthyle.

6. Dispositif selon l'une quelconque des revendications précédentes, dans lequel l'épaisseur du revêtement intermédiaire est d'au moins 10 µm et/ou inférieure à 150 µm.

7. Dispositif selon l'une quelconque des revendications précédentes, dans lequel l'épaisseur du revêtement extérieur est d'au moins 10 µm et/ou inférieure à 250 µm.

8. Dispositif selon l'une quelconque des revendications précédentes, dans lequel le revêtement intermédiaire est en contact direct avec le noyau intérieur, et le revêtement extérieur est en contact direct avec le revêtement intermédiaire.

9. Dispositif selon l'une quelconque des revendications précédentes, dans lequel le dispositif est un anneau intravaginal ou un implant sous-cutané.

10. Dispositif selon l'une quelconque des revendications précédentes, dans lequel la quantité totale de l'au moins un ingrédient pharmaceutique est d'au moins 2 % en poids, de préférence d'au moins 5 % en poids, et/ou est inférieure à 50 % en poids, de préférence inférieure à 30 % en poids, par rapport au poids total du noyau intérieur.

11. Oxybutynine pour utilisation dans le traitement du syndrome de la vessie hyperactive, l'oxybutynine étant délivrée par le dispositif selon l'une quelconque des revendications précédentes.
